# EUROPÄISCHE PATENTSCHRIFT

(11) **EP 3 034 485 B1**
(45) Veröffentlichungstag und Bekanntmachung des Hinweises auf die Patenterteilung: **29.08.2018**
(21) Anmeldenummer: 15200377.8
(22) Anmeldetag: 16.12.2015
(51) Int. Cl.: C07C 1/12, C07C 9/04

(54) **REAKTION VON KOHLENDIOXID MIT WASSERSTOFF UNTER VERWENDUNG EINER PERMSELEKTIVEN MEMBRAN**
REACTION OF CARBON DIOXIDE WITH HYDROGEN USING A PERMSELECTIVE MEMBRANE
RÉACTION DU DIOXIDE DE CARBONE AVEC HYDROGÈNE UTILISANT UNE MEMBRANE PERMSÉLECTIVE

(30) Priorität: 17.12.2014 DE 102014118894
(43) Veröffentlichungstag der Anmeldung: 22.06.2016
(73) Patentinhaber: MUW SCREENTEC Filter- und Präzisionstechnik aus Metall GmbH, 29378 Wittingen (DE)
(72) Erfinder: GRÜTZNER, Jörg, 16552 Schildow (DE); MARTIN, Dirk, 99085 Erfurt (DE)
(74) Vertreter: Oehmke, Volker

(56) Entgegenhaltungen:
- JP-A- 2007 055 970
- OHYA H ET AL: "Methanation of carbon dioxide by using membrane reactor integrated with water vapor permselective membrane and its analysis", JOURNAL OF MEMBRANE SCIENCE, ELSEVIER BV, NL, Bd. 131, Nr. 1-2, 6. August 1997 (1997-08-06), Seiten 237-247, XP004084648, ISSN: 0376-7388, DOI: 10.1016/S0376-7388(97)00055-0

## Beschreibung

Die vorliegende Erfindung betrifft ein Verfahren zur Durchführung von chemischen Gleichgewichtsreaktionen unter Verwendung einer permselektiven Membran. Die Erfindung betrifft insbesondere ein Verfahren zur Umsetzung von Kohlendioxid und Wasserstoff unter Verwendung einer Kohlenstoffmembran.

Die Umsetzung von Wasserstoff mit Kohlendioxid wird im Wesentlichen aus zwei Gründen verfolgt. Einerseits wird zunehmend Wasserstoff durch Elektrolyse hergestellt, um Netzschwankungen aus dem zunehmenden Anteil fluktuierenden Stroms aus regenerativen Quellen zu puffern ("power to gas"). Die Speicherung und die Verteilung von Wasserstoff können zu geringen Anteilen direkt im Erdgasnetz erfolgen. Durch die Umsetzung mit Kohlendioxid zu Methan wären die Speicherung und Verteilung in viel größerem Maßstab möglich. Der zweite Grund betrifft die chemische Bindung und Nutzung von Kohlendioxid und hat damit erhebliche umweit- und klimatechnische Vorteile.

In der dem Fachmann bekannten Sabatier-Reaktion, die eine chemische Gleichgewichtsreaktion ist, werden Kohlendioxid (CO₂) und Wasserstoff (H₂) (Edukte) zu Methan (CH₄) und Wasser (H₂O) (Produkte) umgesetzt. Die Sabatier-Reaktion wird meist bei Drücken von ∼1...3 bara (absoluter Druck; bar absolut), vereinzelt auch bei höheren Drücken von bis zu 15 bara, und in einem Temperaturbereich von rund 200 bis 450 °C durchgeführt. Als geeignete Katalysatoren für diese Reaktion sind beispielsweise Nickel, Ruthenium, Rhodium und Cobalt bekannt, die meist auf einem oxidischen Träger wie SiO₂, TiO₂, MgO, Al₂O₃, La₂O₃ aufgebracht sind, wie dies beispielsweise in Ohya et al. 1997: Methanation of carbon dioxide by using membrane reactor integrated with water vapor permselective membrane and its analysis, Journal of Membrane Science 131 (1-2), beschrieben ist. Am erfolgreichsten stellt sich wohl momentan die Kombination 0,5 % Ru auf TiO₂ dar. Abhängig vom Katalysator können hohe CO₂-Umsätze und CH₄-Ausbeuten bei Temperaturen von ∼300... 350°C erreicht werden (John Ralston (http://www.pennenergy.com/index/power /display/0723256773/articles/pennenergy/ugc/renewable/the-sabatier-reaction.html).

Die Sabatier-Reaktion ist eine sehr anspruchsvolle Reaktion, da sie stark exotherm ist und nach Zugabe der Edukte, je nach Verdünnungsgrad mit inerten Gasen, zu Temperaturen über 600 °C führt. Dieses Temperaturniveau zerstört nicht nur manche Katalysatoren, beispielsweise gehen aktive Zentren verloren, indem z. B. Ni-Partikel im nm-Bereich zusammensintern, sondern verschiebt auch das Reaktionsgleichgewicht in Richtung der Edukte. Zu niedrige Temperaturen haben dagegen kinetische Limitierungen der Reaktion und der Menge der gewünschten Produkte zur Folge.

Verschiedene Publikationen behandeln bereits den Einsatz von Membranen. So führt der Einsatz einer Membran aus 20-fach beschichtetem hydrophilem porösem Glas auf einem keramischen Träger im besten Fall zu einer Erhöhung des CO₂-Umsatzes um 18 % (bei 300°C, 0,2 MPa und Raumgeschwindigkeit = 0,0308 s⁻¹) (Ohya et al.). Die Umsatzerhöhung ist dabei bemerkenswert, allerdings bewegt sich die Raumgeschwindigkeit weit unterhalb industriell notwendiger Bereiche von mindestens 3 s⁻¹. Darüber hinaus werden Membranen vorgelagert zur eigentlichen Sabatier-Reaktion zum Aufkonzentrieren des Kohlendioxids eingesetzt (Hwang et al. 2008: A membrane-based reactive separation system for CO2 removal in a life support system, Journal of Membrane Science 315 (1-2): 116 - 124). Berechnungen zeigen für diesen Fall synergetische Positiveffekte für die Reaktion. Aber auch weitere Prozesskonzepte sind möglich. Über die Fahrweise der Reaktion mit zwei hintereinander geschalteten Reaktoren mit zwischenzeitlichem Auskondensieren des Reaktionswassers kann der hier betrachtete Wasserstoffumsatz von ca. 90 % auf bis zu 99 % gesteigert werden (Habazaki et al. 1998: Co-methanation of carbon monoxide and carbon dioxide on supported nickel and cobalt catalysts prepared from amorphous alloys, Applied Catalysis A: General 172 (1): 131 - 140). Die Trennung der Produkte Methan und Wasser ist für eine mögliche Einspeisung in das Erdgasnetz aber eine zwingende Vorrausetzung.

Da es sich bei der Sabatier-Reaktion um eine exotherme Gleichgewichtsreaktion handelt, ist sie mit zunehmender Reaktions- bzw. Prozesstemperatur ausbeutelimitiert. Der Entzug eines Reaktionsproduktes direkt aus der Reaktion im Prozess bewirkt eine Erhöhung der Triebkraft zur Produktbildung. Eine geeignete wasserabtrennende Membran bewirkt genau dies, indem das Reaktionsprodukt Wasser direkt im Prozess abgetrennt wird. So wird eine Ausbeutesteigerung verglichen zum Prozess ohne Membran erreicht. Weiterhin wird durch die Abtrennung des Reaktionswassers eine nachgeordnete Trocknung des Methans zur Erreichung der Einspeisequalität überflüssig und dieser Prozessschritt kann eingespart werden.

Methanol kann beispielsweise aus einem Synthesegas aus Kohlenmonoxid und Wasserstoff hergestellt werden. Insbesondere bei der Herstellung von Methanol aus Kohlendioxid und Wasserstoff als Edukte und Methanol und Wasser als Produkte einer chemischen Gleichgewichtsreaktion kann die Ausbeute ebenfalls verbessert werden, wenn es gelingt, das Wasser auf der Seite der Produkte abzutrennen (JP 2007055970 A).

Zur Abtrennung von Wasser aus Gasgemischen bei höherer Temperatur werden üblicherweise hydrophile Membranen eingesetzt. Dies sind insbesondere SiO₂-Membranen und Zeolith-A-Membranen (Wang et al., 2011: "Effects of water vapor on gas permeation and separation properties of MFI zeolite membranes at high temperatures, AIChE Journal 58(1); Sano et al., 1994: "Separation of ethanol/water mixture by silicalite membrane on pervaporation, Journal of Membrane Science 95 (3); Hamzah et al., 2013: Pervaporation through NaA zeolite membranes - A review", The Third Basic Science International Conference 2013).

Bevorzugte Anwendung ist die Entwässerung und Trocknung von Lösemitteldämpfen. Der Einsatz dieser Membranen in der Sabatier-Reaktion zur Abtrennung von Wasser aus einem Gemisch mit Wasserstoff, Kohlendioxid und Methan zeigt jedoch eine unzureichende Selektivität und des Weiteren eine zu geringe Stabilität der Materialien unter den notwendigen Bedingungen der Sabatier-Reaktion. SiO₂-Membranen unterliegen bei reduzierenden Bedingungen, wie sie in der Sabatier-Reaktion vorliegen, oberhalb von 100 °C thermischer Degradation (Damle et al., 1995: "Thermal and chemical degradation of inorganic membrane materials", Technical Report; Gu et al.: "Hydrothermally stable silica-alumina composite membranes for hydrogen separation", Journal of Membrane Science 310 (1-2): 28 - 37) und Zeolith-A-Membranen besitzen mangelnde Stabilität gegenüber sauren Milieus und teilweise limitierte thermische Stabilität (Hamzah et al., 2013: Pervaporation through NaA Zeolite Membranes - A Review, The Third Basic Science International Conference; C03; Caro et al., 2009: Why is it so extremely difficult to prepare shapeselective Al-rich zeolite membranes like LTA and FAU for gas separation?, Separation and Purification Technology 66(1): 143 - 147), wie sie in Kombination von H₂O mit CO₂ auftreten.

Der Erfindung liegt die Aufgabe zugrunde, eine Möglichkeit zur Verfahrensführung von Gleichgewichtsreaktionen vorzuschlagen, bei denen auf der Seite der Reaktionsprodukte Wasser abgetrennt wird und eine verbesserte Ausbeute an Reaktionsprodukten erzielt wird.

Die Aufgabe wird durch den Gegenstand des Anspruchs 1 gelöst. Vorteilhafte Ausgestaltungen sind in den abhängigen Ansprüchen angegeben.

Die Aufgabe wird durch ein Verfahren zur Durchführung von chemischen Gleichgewichtsreaktionen, bei denen Kohlendioxid und Wasserstoff umgesetzt werden, unter Verwendung einer permselektiven Membran gelöst, wobei es sich bei der Gleichgewichtsreaktion entweder um eine Sabatier-Reaktion oder eine Methanol-Herstellungsreaktion handelt, und wobei durch Wirkung der permselektiven Membran Mengenanteile wenigstens eines bei der Reaktion erhaltenen Produkts abgetrennt und der Reaktion entzogen werden. Kennzeichnend für ein erfindungsgemäßes Verfahren ist, dass eine permselektive Membran verwendet wird, die aus einem Material besteht, das eine poröse Struktur mit mittleren Porengrößen von weniger als 0,45 nm (4,5 Ä) aufweist und deren Material wenigstens auf seinen freien Oberflächen hydrophob ist. Als hydrophob wird ein Material angesehen, wenn an einem aufgesetzten Wassertropfen Benetzungswinkel von 85° und darüber gemessen werden. Die chemische Gleichgewichtsreaktion wird unter Anwesenheit eines wasserhaltigen Gasgemisches durchgeführt. Ein wasserhaltiges Gasgemisch ist beispielsweise durch die Produkte der Sabatier-Reaktion Methan und Wasser erhalten. Durch die hohen Temperaturen, bei denen diese Gleichgewichtsreaktion erfolgt, liegt das Wasser als Wasserdampf vor. Eine Mischung von Methan und Wasserdampf wird im Sinne dieser Beschreibung als Gasgemisch, insbesondere als wasserhaltiges Gasgemisch verstanden.

Kern der Erfindung ist die Verwendung einer im Wesentlichen aus Kohlenstoff und / oder aus Kohlenwasserstoffen aufgebauten organischen Membran. Vorzugsweise ist die permselektive Membran (fortan auch kurz: Membran) eine Kohlenstoffmembran.

Dabei hat sich überraschend gezeigt, dass durch eine solche Kohlenstoffmembran unter den Bedingungen der Gleichgewichtsreaktion (fortan auch kurz: Reaktion) selektiv Wasser aus dem genannten Gemisch der Produkte abgetrennt wird.

Die Membran kann dabei beispielsweise auf keramischen Elementen verschiedener Länge und Durchmesser aufgebracht sein, die als Träger fungieren. Die Membranen können auf einer Innenseite und / oder auf einer Außenseite der Träger vorhanden sein. Die Träger können auf der Innenseite und / oder auf der Außenseite Zwischenschichten mit abnehmender Porengröße aufweisen, über denen die Membran angeordnet sein kann. Träger weisen beispielsweise eine Länge von 10 bis 2000 mm, beispielsweise 105 mm und 250 mm, auf. Die Träger können auf ihrer Innenseite und / oder auf ihrer Außenseite teilweise oder vollständig mit der Membran beschichtet sein. Weiterhin können Membranen auf beliebig geformten Trägern von beispielsweise 10 bis 2000 mm, beispielsweise von 500 mm, Länge hergestellt sein. Als zusätzlicher Schritt zur Außenbeschichtung können einseitig verschlossene Keramikträger hergestellt werden und die Membran kann auch auf diesen außenseitig appliziert werden.

Bei einer Verwendung teilweise beschichteter Träger ist beispielsweise durch eine konstruktive Maßnahme dafür Sorge zu tragen, dass ein Abtrennen des Wassers nur über die Bereiche des Trägers erfolgt, die von der Membran bedeckt sind.

Es ist daher eine bevorzugte Ausführung des erfindungsgemäßen Verfahrens, wenn die permselektive Membran eine Kohlenstoffmembran ist, die Struktur des Materials graphitähnlich und durch Schichten des Materials gebildet ist, die Struktur durch wenigstens eine Folge der Schichten gebildet ist, wobei die Schichten in Ebenen angeordnet vorliegen, zwischen benachbarten Schichten ein mittlerer Abstand der Schichten von weniger als 0,45 nm (4,5 Ä) vorliegt und die Folge der Schichten turbostratisch fehlgeordnet ist.

In einer sehr günstigen Ausführung des erfindungsgemäßen Verfahrens ist eine Membran verwendet, bei der wenigstens ein metallhaltiger Katalysator auf einer Oberfläche der permselektiven Membran aufgebracht ist. Dabei bedeutet der Begriff "aufgebracht", dass der Katalysator mit der Membran verbunden ist oder mit dieser in direktem Kontakt steht. Vorzugsweise ist der Katalysator in Form von Partikeln auf einer Oberfläche der Membran gebunden und / oder liegt dieser als Schüttgut an oder auf. Der Katalysator kann auch als eine Beschichtung auf der Membran oder auf Teilen der Membran vorhanden sein.

Katalysatoren können dabei metallhaltige Katalysatoren aus einer Gruppe, beinhaltend die Metalle Ruthenium, Nickel, Cobalt, Rhodium, Platin und Palladium oder deren Legierungen, sein. Durch den Begriff der metallhaltigen Katalysatoren sind auch metallische Katalysatoren umfasst.

Sehr günstig für die Reaktionsführung und eine hohe Produktausbeute ist es, wenn die chemische Gleichgewichtsreaktion bei einem Wasserstoff-Überschuss bezogen auf den stöchiometrischen Einsatz molekularen Wasserstoffs (H₂) durchgeführt wird. So kann der Wasserstoff-Überschuss bezogen auf den stöchiometrischen Einsatz molekularen Wasserstoffs 2,5 % betragen. Durch einen Überschuss an Wasserstoff wird eine unerwünschte Koksbildung weitgehend vermieden.

Als Quelle des molekularen Wasserstoffs können vorteilhaft Gase, wie Biogas, Klärgas, Biomethan, molekularen Wasserstoff enthaltende Kohlendioxidströme, molekularen Wasserstoff freisetzende Elektrolysen, Wasserstoffströme oder Kombinationen dieser Quellen, verwendet werden.

Es ist dabei für eine effiziente Verfahrensdurchführung vorteilhaft, wenn die Quellen molekularen Wasserstoffs vor deren Verwendung in dem erfindungsgemäßen Verfahren verfahrenstechnisch behandelt werden. Sie können beispielsweise reduziert und entschwefelt werden, so dass diese ≤ 1 ppm Sauerstoff (02) und Schwefel (S) enthalten und eine Einspeisequalität sichergestellt wird.

Die chemische Gleichgewichtsreaktion ist vorzugsweise entweder eine Sabatier-Reaktion oder eine Methanol-Herstellungsreaktion.

Das erfindungsgemäße Verfahren, insbesondere die Sabatier-Reaktion, wird vorzugsweise bei einem Druck, ausgewählt aus einem Bereich von 1 bis einschließlich 100 bar, z. B. 5, 75, 100 bar, und bei einer Temperatur, ausgewählt aus einem Bereich von 150 bis einschließlich 600 °C, z. B. 400, 500 °C, durchgeführt.

Es hat sich als günstig erwiesen, dass die Sabatier-Reaktion bei einem Druck, ausgewählt aus einem Bereich von 10 bis 20 bar, und bei einer Temperatur, ausgewählt aus einem Bereich von 250 bis 450 °C, durchgeführt wird. Insbesondere kann die Sabatier-Reaktion effizient bei einem Druck von 20 bar und bei einer Temperatur von 300 °C durchgeführt werden.

Ist die chemische Gleichgewichtsreaktion eine Methanol-Herstellungsreaktion, wird diese vorzugsweise bei einem Druck ausgewählt aus einem Bereich von 80 bis einschließlich 250 bar und bei einer Temperatur ausgewählt aus einem Bereich von 150 bis einschließlich 400° C durchgeführt.

Methanol (CH₃OH) ist als einfachster Vertreter der Stoffgruppe der Alkohole eine der meisthergestellten organischen Chemikalien und dient als Ausgangsstoff vieler weiterer chemischer Produkte (z. B. Formaldehyd, Ameisensäure usw.). Die technische Herstellung erfolgt hauptsächlich durch eine katalytisch unterstützte Reaktion von CO und H₂. Die Verfahren zur Methanol-Herstellung werden nach den vorherrschenden Reaktionsdrücken in Hochdruckverfahren (250-350 bar, ∼370 °C), Mitteldruckverfahren (100-250 bar, ∼220-300 °C) und Niederdruckverfahren (50-100 bar, 200-300 °C) unterteilt. Aufgrund ökonomischer Nachteile wird das Hochdruckverfahren heutzutage nicht mehr angewendet. Es werden im Stand der Technik weitestgehend Kupfer-Zink-Katalysatoren auf Aluminiumoxid genutzt.

Eine alternative Variante zur Herstellung von CH₃OH ist die Reaktion von CO₂ mit H₂, um auch bei der Synthese dieser Grundchemikalie auf das bisherige Abfallprodukt CO₂ zurückgreifen zu können. Bei dieser Reaktion entsteht neben CH₃OH auch H₂O. Im Vergleich zur Erzeugung von Methanol aus Synthesegas liegt die Produktivität bei der Erzeugung aus CO₂ und H₂ um den Faktor 3-10 geringer, da das Wasser die Reaktion behindert. Allerdings kann mit einer kontinuierlichen Abführung des Reaktionsproduktes Wasser die Ausbeute erhöht werden. Dies wird durch den Einsatz der Membranen erreicht.

Die Membranen können in flexibler Geometrie verwendet werden. Die Membranen erlauben eine selektive Abtrennung von Wasser unter Prozessbedingungen. Ausgestattet mit einem für die durchzuführende chemische Gleichgewichtsreaktion aktiven Katalysator kann die Ausbeute signifikant erhöht und die Erreichung der Einspeisequalität erleichtert werden.

Durch ein erfindungsgemäßes Verfahren unter Verwendung organischer Membranen mit hydrophoben Oberflächen wird vorteilhaft selbst bei hohen Temperaturen selektiv Wasser aus der Reaktion abtrennt. Ein verwendeter Katalysator kann dabei als Schüttgut auf der Membran angeordnet sein oder es wird eine katalytisch aktive Membran verwendet. Bevorzugter Einsatz der Membran ist die Umsetzung von Kohlendioxid und Wasserstoff zu Methan (Sabatier-Reaktion) oder Methanol.

Die erfindungsgemäße Verwendung der beschriebenen Membranen wird beispielsweise zur Wasserabtrennung aus der Sabatier-Reaktion genutzt. Dazu ist ein direkter Kontakt zwischen wasserabtrennender Membran und Katalysator sinnvoll. Die trennaktive Membran kann als Katalysatorträger dienen. Der Katalysator oder ein Katalysatorvorläufer wird auf die trennaktive Schicht aufgebracht und gegebenenfalls in geeigneter Form, beispielsweise durch Wärmezufuhr, aktiviert. Die Aktivierung des Katalysators erfolgt beispielsweise unter Reaktionsbedingungen der Sabatier-Reaktion.

Die erfindungsgemäße Verwendung der beschriebenen Membranen erlaubt es außerdem, effizient eine chemische Gleichgewichtsreaktion durchzuführen, bei der aus Kohlendioxid und Wasserstoff auf der Eduktseite Methanol und Wasser auf der Produktseite gewonnen werden. Durch die selektive Abtrennung von Wasser wird das Gleichgewicht in Richtung auf die Produkte verschoben und die Bildung von Methanol begünstigt.

Für das erfindungsgemäße Verfahren werden nachfolgend Ausführungsbeispiele angegeben.

### 1. Reaktion ohne Membran:

Als Katalysator dient ein Aluminium-unterstütztes Nickeloxid, beispielsweise das kommerziell verfügbare Produkt METH 134. Der Katalysator wurde als Schüttung appliziert und in einem Wasserstoff-/Stickstoffstrom (1:9) entsprechend aktiviert. Der Eduktvolumenstrom beträgt insgesamt ca. 250 Nl/h (Normliter je Stunde). Das H₂/CO₂-Verhältnis beträgt 4,1:1 (19,6 Vol% CO₂, 80,4 Vol% H₂) bei einem Prozessdruck von 20 barÜ (20 bar Überdruck) und Reaktionstemperaturen von 275 - 450 °C. Es werden Umsätze zwischen 85 und 89 % bei einer CH₄-Selektivität von > 99 % erreicht. Die CH₄-Ausbeute beträgt ebenfalls zwischen 85 und 89 %.

### 2. Reaktion mit Membran

Als Katalysator dient ein Aluminium-unterstütztes Nickeloxid, beispielsweise das kommerziell verfügbare Produkt METH 134 (Hersteller Clariant). Der Katalysator wurde als Schüttung appliziert und in einem Wasserstoff-/Stickstoffstrom (1:9) entsprechend aktiviert. Der Eduktvolumenstrom beträgt insgesamt ca. 250 Nl/h. Das H₂/CO₂-Verhältnis beträgt 4,1:1 (19,6 Vol% CO₂, 80,4 Vol% H₂) bei einem Prozessdruck von 20 barÜ und Reaktionstemperaturen von 240 - 450 °C. Die Raumgeschwindigkeit an der Membran beträgt ∼21,5 s⁻¹ bzw. entspricht einer Lineargeschwindigkeit von ∼0,04 m/s. Es werden Umsätze > 90 % bei einer CH₄-Selektivität von > 99 % erreicht. Die CH₄-Ausbeute beträgt ebenfalls > 90 %.

### 3. Reaktion mit Membran (katalysatorbeschichtet)

Als Katalysatorvorläufer wird eine Beschichtung der Membran mit Nickeloxid verwendet. Dieser Vorläufer wird in einem Wasserstoff-/Stickstoffstrom (1:9) zum aktiven metallischen Nickel reduziert und entsprechend aktiviert. Der Eduktvolumenstrom beträgt insgesamt ca. 1000 Nl/h. Das H₂/CO₂-Verhältnis beträgt 4,1:1 (19,6 Vol% CO₂, 80,4 Vol% H₂) bei einem Prozessdruck von 20 barÜ und Reaktionstemperaturen von 240 - 450 °C. Die Raumgeschwindigkeit an der Membran beträgt ∼58 s⁻¹ bzw. entspricht einer Lineargeschwindigkeit von ∼0,02 m/s. Es werden Umsätze > 95 % bei einer CH₄-Selektivität von > 99 % erreicht. Die CH₄-Ausbeute beträgt ebenfalls > 95 %.

## Patentansprüche

1. Verfahren zur Durchführung von chemischen Gleichgewichtsreaktionen, bei denen Kohlendioxid und Wasserstoff umgesetzt werden, unter Verwendung einer permselektiven Membran, wobei durch Wirkung der permselektiven Membran Mengenanteile wenigstens Wasser als eines bei der Reaktion erhaltenen Produkte abgetrennt und der Reaktion entzogen werden, **dadurch gekennzeichnet, dass** die Gleichgewichtsreaktion entweder eine Sabatier-Reaktion oder eine Methanol-Herstellungsreaktion ist und eine permselektive Membran verwendet wird, die aus einem wenigstens Kohlenstoff und / oder Kohlenwasserstoffe enthaltendem Material besteht, das eine poröse Struktur mit mittleren Porengrößen von weniger als 0,45 nm (4,5 Ä) aufweist und deren Material wenigstens auf seinen freien Oberflächen hydrophob ist.

2. Verfahren nach Anspruch 1, **dadurch gekennzeichnet, dass**
- die permselektive Membran eine Kohlenstoffmembran ist,
- die Struktur des Materials graphitähnlich und durch Schichten des Materials gebildet ist,
- die Struktur durch wenigstens eine Folge der Schichten gebildet ist, wobei die Schichten in Ebenen angeordnet vorliegen,
- zwischen benachbarten Schichten ein mittlerer Abstand der Schichten von weniger als 0,45 nm (4,5 Ä) vorliegt und
- die Folge der Schichten turbostratisch fehlgeordnet ist.

3. Verfahren nach einem der Ansprüche 1 oder 2, **dadurch gekennzeichnet, dass** wenigstens ein metallhaltiger Katalysator auf einer Oberfläche der permselektiven Membran aufgebracht ist.

4. Verfahren nach Anspruch 3, **dadurch gekennzeichnet, dass** metallhaltige Katalysatoren aus einer Gruppe, beinhaltend die Metalle Ruthenium, Nickel, Kobalt, Rhodium, Platin und Palladium oder deren Legierungen, ausgewählt sind.

5. Verfahren nach einem der vorhergehenden Ansprüche, **dadurch gekennzeichnet, dass** die chemische Gleichgewichtsreaktion bei einem Wasserstoff-Überschuss bezogen auf den stöchiometrischen Einsatz molekularen Wasserstoffs durchgeführt wird.

6. Verfahren nach Anspruch 5, **dadurch gekennzeichnet, dass** der Wasserstoff-Überschuss bezogen auf den stöchiometrischen Einsatz molekularen Wasserstoffs 2,5% beträgt.

7. Verfahren nach einem der vorhergehenden Ansprüche, **dadurch gekennzeichnet, dass** als Quelle molekularen Wasserstoffs Biogas, Klärgas, Biomethan, molekularen Wasserstoff enthaltende Kohlendioxidströme, molekularen Wasserstoff freisetzende Elektrolysen, Wasserstoffströme oder Kombinationen dieser Quellen verwendet werden.

8. Verfahren nach Anspruch 7, **dadurch gekennzeichnet, dass** die Quellen molekularen Wasserstoffs reduziert und entschwefelt werden und ≤ 1 ppm Sauerstoff (02) und Schwefel (S) enthalten.

9. Verfahren nach Anspruch 1, **dadurch gekennzeichnet, dass** die chemische Gleichgewichtsreaktion die Sabatier-Reaktion ist und diese bei einem Druck ausgewählt aus einem Bereich von 1 bis 100 bar und bei einer Temperatur ausgewählt aus einem Bereich von 150 bis 600 °C durchgeführt wird.

10. Verfahren nach Anspruch 9, **dadurch gekennzeichnet, dass** die Sabatier-Reaktion bei einem Druck ausgewählt aus einem Bereich von 10 bis 20 bar und bei einer Temperatur ausgewählt aus einem Bereich von 250 bis 450 °C durchgeführt wird.

11. Verfahren nach Anspruch 10, **dadurch gekennzeichnet, dass** die Sabatier-Reaktion bei einem Druck von 20 bar und bei einer Temperatur von 300°C durchgeführt wird.

12. Verfahren nach Anspruch 1, **dadurch gekennzeichnet, dass** die chemische Gleichgewichtsreaktion eine Methanol-Herstellungsreaktion ist und bei einem Druck ausgewählt aus einem Bereich von 80 bis einschließlich 250 bar und bei einer Temperatur ausgewählt aus einem Bereich von 150 bis einschließlich 400° C durchgeführt wird.

## Claims

1. Method for carrying out chemical equilibrium reactions in which carbon dioxide and hydrogen are converted, using a permselective membrane, wherein quantitative proportions of at least water as one of products obtained in the reaction are separated and removed from the reaction through the action of the permselective membrane, **characterized in that** the equilibrium reaction is either a Sabatier reaction or a methanol production reaction and a permselective membrane is used which is made from a material containing at least carbon and / or hydrocarbons, said material having a porous structure with mean pore sizes of less than 0.45 nm (4.5 Å) and being hydrophobic at least on the free surfaces thereof.

2. Method according to claim 1, **characterized in that**
- the permselective membrane is a carbon membrane,
- the structure of the material is graphite-like and is formed by layers of the material,
- the structure is formed by at least one series of layers, wherein the layers are arranged in planes,
- there is a mean spacing of the layers of less than 0.45 nm (4.5 Å) between adjacent layers, and
- the series of layers is turbostratically disordered.

3. Method according to one of claims 1 or 2, **characterized in that** at least one metal-containing catalyst is arranged on a surface of the permselective membrane.

4. Method according to claim 3, **characterized in that** metal-containing catalysts are selected from a group comprising the metals ruthenium, nickel, cobalt, rhodium, platinum and palladium, or alloys thereof.

5. Method according to one of the preceding claims, **characterized in that** the chemical equilibrium reaction is carried out with a hydrogen surplus with respect to the stoichiometric use of molecular hydrogen.

6. Method according to claim 5, **characterized in that** the hydrogen surplus amounts to 2.5% with respect to the stoichiometric use of molecular hydrogen.

7. Method according to one of the preceding claims, **characterized in that** biogas, sewage gas, biomethane, molecular hydrogen-containing carbon dioxide streams, molecular hydrogen-releasing electrolysis, hydrogen streams or combinations thereof are used as sources of molecular hydrogen.

8. Method according to claim 7, **characterized in that** the sources of molecular hydrogen are reduced and desulfurized and contain ≤ 1 ppm of oxygen (O₂) and sulfur (S).

9. Method according to claim 1, **characterized in that** the chemical equilibrium reaction is the Sabatier reaction, and this Sabatier reaction is carried out at a pressure selected from a range of 1 to 100 bar and at a temperature selected from a range of 150 to 600 °C.

10. Method according to claim 9, **characterized in that** the Sabatier reaction is carried out at a pressure selected from a range of 10 to 20 bar and at a temperature selected from a range of 250 to 450 °C.

11. Method according to claim 10, **characterized in that** the Sabatier reaction is carried out at a pressure of 20 bar and at a temperature of 300 °C.

12. Method according to claim 1, **characterized in that** the chemical equilibrium reaction is a methanol production reaction and is carried out at a pressure selected from a range of 80 bar up to and including 250 bar and at a temperature selected from a range of 150 °C up to and including 400 °C.

## Revendications

1. Procédé pour réaliser des réactions d'équilibre chimique dans lesquelles du dioxyde de carbone et de l'hydrogène sont convertis, en utilisant une membrane permsélective, des proportions quantitatives au moins d'eau comme un de produits obtenus de la réaction étant séparées et retirées de la réaction par l'action de la membrane permsélective, **caractérisé en ce que** la réaction d'équilibre est soit une réaction de Sabatier, soit une réaction de production de méthanol, et que une membrane permsélective est utilisée qui consiste en un matériau contenant au moins du carbone et / ou des hydrocarbures, le matériau ayant une structure poreuse avec des tailles moyennes de pores inférieures à 0,45 nm (4,5 Å) et étant hydrophobe au moins sur ses surfaces libres.

2. Procédé selon la revendication 1, **caractérisé en ce que**
- la membrane permsélective est une membrane de carbone,
- la structure du matériau est de type graphite et formée par des couches du matériau,
- la structure est formée par au moins une séquence de couches, les couches étant disposées dans des plans,
- il y a un espacement moyen des couches inférieur à 0,45 nm (4,5 Å) entre des couches adjacentes, et
- la séquence des couches est désordonnée turbostratiquement.

3. Procédé selon une des revendications 1 ou 2, **caractérisé en ce qu'**au moins un catalyseur contenant du métal est appliqué sur une surface de la membrane permsélective.

4. Procédé selon la revendication 3, **caractérisé en ce que** les catalyseurs contenant du métal sont sélectionnés d'un groupe comprenant les métaux ruthénium, nickel, cobalt, rhodium, platine et palladium ou leurs alliages.

5. Procédé selon une des revendications précédentes, **caractérisé en ce que** la réaction d'équilibre chimique est réalisée à un excès d'hydrogène par rapport à l'utilisation stoechiométrique d'hydrogène moléculaire.

6. Procédé selon la revendication 5, **caractérisé en ce que** l'excès d'hydrogène est 2,5% par rapport à l'utilisation stoechiométrique d'hydrogène moléculaire.

7. Procédé selon une des revendications précédentes, **caractérisé en ce que** du biogaz, du gaz d'égout, du biométhane, des courants de dioxyde de carbone contenant de l'hydrogène moléculaire, d'électrolyses dégageant de l'hydrogène moléculaire, des courants d'hydrogène ou des combinaisons de ceux-ci sont utilisés comme source de l'hydrogène moléculaire.

8. Procédé selon la revendication 7, **caractérisé en ce que** les sources d'hydrogène moléculaire sont réduites et désulfurées et contiennent ≤ 1 ppm d'oxygène (O₂) et de soufre (S).

9. Procédé selon la revendication 1, **caractérisé en ce que** la réaction d'équilibre chimique est la réaction de Sabatier et celle-ci est réalisée à une pression choisie d'une gamme de 1 à 100 bars et à une température choisie d'une gamme de 150 à 600 °C.

10. Procédé selon la revendication 9, **caractérisé en ce que** la réaction de Sabatier est réalisée à une pression choisie d'une gamme de 10 à 20 bars et à une température choisie d'une gamme de 250 à 450 °C.

11. Procédé selon la revendication 10, **caractérisé en ce que** la réaction de Sabatier est réalisée à une pression de 20 bars et à une température de 300 °C.

12. Procédé selon la revendication 1, **caractérisé en ce que** la réaction d'équilibre chimique est une réaction de production de méthanol et est réalisée à une pression choisie d'une gamme de 80 jusqu'à 250 bars inclus et à une température choisie d'une gamme de 150 jusqu'à 400 °C inclus.
